# EUROPEAN PATENT APPLICATION

(11) **EP 3 868 897 A1**
(43) Date of publication of application: **25.08.2021**
(21) Application number: 20305161.0
(22) Date of filing: 20.02.2020
(51) Int. Cl.: C12Q 1/6886

(54) **METHOD FOR IMPROVING THE TREATMENT WITH IMMUNE CHECKPOINT BLOCKADE THERAPY**

(71) Applicant: Worldwide Innovative Network, 94800 Villejuif (FR)
(72) Inventor: LAZAR, Vladimir, 94800 VILLEJUIF (FR)
(74) Representative: Cabinet Becker et Associés

(57) **Abstract**

The present invention relates to a method for determining if a subject having a cancer is susceptible to have a therapeutic benefit of a treatment with an immune checkpoint blockade therapy.

## Description

### FIELD OF THE INVENTION

The present invention relates to the field on oncology, especially to personalized medicine in cancer therapy, especially by an immune checkpoint blockade therapy.

### BACKGROUND OF THE INVENTION

Immunotherapy represents today a major field of interest in the treatment of cancers. Indeed, leverage of the negative immune checkpoint blockade is able to induce durable responses across multiple types of cancers. The most advanced knowledge has been generated around the therapies targeting PD-1/PD-L1 or CTLA-4, and to a less extent anti-LAG3 or anti-TLR4. However, overall, only a fraction of patients has a therapeutic benefit of treatments targeting the immune checkpoint blockade, more specifically less than 25% of them and only some of them has responses with long duration. Indeed, most patients do not respond or, after an initial response, develop resistance. Many patients have also important toxicities problems due to the treatment. Therefore, identifying those patients that would have a clinical benefit remains an important unmet need in the field.

Many biomarkers have been used in the past years aiming to achieve this goal. However, to some extent, biomarkers strategies were biased. Drug developers oriented the research to biomarkers predictive to the response to immunotherapies only to deal with their own therapies. For example, anti-PD-Ll or anti-PD-1 therapies only took into account the level of PD-L1, the tumor mutations burden (an increased number of mutations being supposed to generate an increased amount of neoantigens derived from mutated proteins and recognized as "non-self", or microsatellite instability reflecting a particular profile of mutations with high number of mutations). Nevertheless, most of them did not meet expectations and failed to correctly predict the efficacy of the immunotherapies treatments.

By consequences, immunotherapies are currently limited to a minority of patient and indications. Only 25 % of patients have a response and among them, only a fraction has long durable responses. Therefore, there is a strong need to methods allowing an effective selection of the patients that would have a therapeutic benefit of an immune checkpoint blockade therapy.

### SUMMARY OF THE INVENTION

The inventors present a novel concept of biomarker assessment that does not consider only the target but the global context of the immune blockade that can be targeted by immune checkpoint therapies. The method proposed by the inventors provide useful information that simultaneously and globally analyzes the key players of the immune-negative blockade. The novel biomarker strategy is made possible by simultaneous analysis of the tumor tissue and the analogous histologically matched normal tissue from the same patient. The method of the present invention enables to analyze individually each key players of immune blockade, but most importantly can assess their complex interactions.

Accordingly, the present disclosure provides a method for determining if a subject suffering from a cancer is susceptible to have a therapeutic benefit of a treatment with an immune checkpoint blockade therapy, comprising
- providing mRNA expression level in a tumor sample and a normal histologically matched sample of a set of genes comprising genes of one or more of the following groups of genes: 1) PD-1, PD-L1, and PD-L2; 2) CTLA-4, CD80, CD86, and CD28; 3) LAG3; 4) TLR4 and 5) CD8, CD16 and FOXP3; the set of genes comprising at least the genes of the group 1) or 2); and wherein the tumor and normal histologically matched samples are from the same subject; the mRNA expression level being optionally corrected with the expression of miRNA targeting the transcript;
- classifying the genes into three classes: i) a first class in which genes are overexpressed in the tumor sample in comparison to the normal histologically matched sample; ii) a second class in which gene are expressed at a similar level in the tumor sample in comparison to the normal histologically matched sample; and iii) a third class in which genes are underexpressed in the tumor sample in comparison to the normal histologically matched sample; and
- displaying the genes classified into the three classes.

In a particular aspect, the set of genes comprises at least the genes of the group 1) and 2).

In another particular aspect, the set of genes is selected in one of the following sets: a) PD-1, PD-L1, PD-L2, CTLA-4, CD80, CD86, CD28, LAG3, TLR4, CD8, CD16 and FOXP3; b) PD-1, PD-L1, PD-L2, CTLA-4, CD80, CD86, LAG3 and TLR4; c) PD-1, PD-L1, PD-L2, CTLA-4, CD80, CD28, CD8, CD16 and FOXP3; d) PD-1, PD-L1, PD-L2, LAG3, TLR4, CD8, CD16 and FOXP3; and e) CTLA-4, CD80, CD86, CD28, LAG3, TLR4, CD8, CD16 and FOXP3.

In an aspect of the method, mRNA expression level of other genes is studied in the method but the total number of genes is no more than 15 genes.

Optionally, a gene is overexpressed when the fold change between the tumor sample and the normal histologically matched sample is higher than 1.3, a gene is expressed at a similar level when the fold change is between -1.3 and 1.3, and a gene is underexpressed when the fold change is lower than -1.3.

Optionally, the genes classified into the three classes are displayed as a graph, preferably a graph showing the expression intensity in the tumor sample on the ordinate and the expression intensity in the normal histologically matched sample on the abscissa. For instance, the genes of each of the three classes can be shown with a distinct mode between each other's and optionally the genes of each of the groups can also be shown with a distinct mode between each other's.

Optionally, the susceptibility to have a therapeutic benefit of a treatment with an immune checkpoint blockade therapy is assessed based on the presence of genes of the groups in one of the three classes and optionally the expression intensity of the genes of the groups in the tumor sample.

Optionally, the immune checkpoint blockade therapy is selected from the group consisting an anti-PD-1 antibody, an anti-PD-L1 antibody, an anti-CTLA-4 antibody, an anti-LAG3 antibody and any combination thereof.

The present disclosure also provides a method for selecting a set of genes for which the differential expression between a tumor sample and a normal histologically matched sample from the same patient is indicative of a therapeutic benefit of a treatment with an immune checkpoint blockade therapy, wherein the method comprises
- providing, for several patients having a cancer and being treated with the same immune checkpoint blockade therapy, mRNA expression level in a tumor sample and a normal histologically matched sample of a set of genes comprising genes of one or more of the following groups of genes: 1) PD-1, PD-L1, and PD-L2; 2) CTLA-4, CD80, CD86, and CD28; 3) LAG3; 4) TLR4 and 5) CD8, CD16 and FOXP3; the set of genes comprising at least the genes of the group 1) or 2); and wherein the tumor and normal histologically matched samples are from the same subject; the mRNA expression level being optionally corrected with the expression of miRNA targeting the transcript;
- determining the correlation for the differential expression of combinations of 4, 5, 6, 7, 8, 9 and/or 10 genes of the set of genes; and,
- selecting the combination of genes associated with the best correlation to therapeutic benefit of a treatment with an immune checkpoint blockade therapy.

In an aspect of the method, the set of genes is selected in one of the following sets: a) PD-1, PD-L1, PD-L2, CTLA-4, CD80, CD86, CD28, LAG3, TLR4, CD8, CD16 and FOXP3; b) PD-1, PD-L1, PD-L2, CTLA-4, CD80, CD86, LAG3 and TLR4; c) PD-1, PD-L1, PD-L2, CTLA-4, CD80, CD28, CD8, CD16 and FOXP3; d) PD-1, PD-L1, PD-L2, LAG3, TLR4, CD8, CD16 and FOXP3; and e) CTLA-4, CD80, CD86, CD28, LAG3, TLR4, CD8, CD16 and FOXP3, preferably PD-1, PD-L1, PD-L2, CTLA-4, CD80, CD86, CD28, LAG3, TLR4, CD8, CD16 and FOXP3.

Optionally, mRNA expression level of other genes is studied in the method but the total number of genes is no more than 15 genes.

Optionally, the immune checkpoint blockade therapy is selected from the group consisting an anti-PD-1 antibody, an anti-PD-L1 antibody, an anti-CTLA-4 antibody, an anti-LAG3 antibody and any combination thereof.

In addition, the present disclosure provides a method for determining if a subject suffering from a cancer is susceptible to have a therapeutic benefit of a treatment with an immune checkpoint blockade therapy targeting CTLA-4, especially an anti-CTLA-4 antibody, wherein the expression level of a set of genes comprising or consisting of PD-1, PD-L1, CD80, CD28, LAG3, TLR4, CD8, CD16 and FOXP3 is determined in a tumor sample and a normal histologically matched sample from the patient, and the susceptibility for having a therapeutic benefit of a treatment with the immune checkpoint blockade therapy targeting CTLA-4, especially an anti-CTLA-4 antibody, is inversely correlated with the fold change of the expression for the set of genes.

The present disclosure also provides a method for determining if a subject suffering from a cancer is susceptible to have a therapeutic benefit of a treatment with an immune checkpoint blockade therapy targeting PD-1/PD-L1, especially an anti-PD-1 or anti-PD-Ll antibody, wherein the expression level of a set of genes comprising or consisting of PD-L2, CTLA-4, LAG3, TLR4 and FOXP3 is determined in a tumor sample and a normal histologically matched sample from the patient, and the susceptibility for having a therapeutic benefit of a treatment with the immune checkpoint blockade therapy targeting PD-1/PD-L1, especially an anti-PD-1 or anti-PD-Ll antibody, is inversely correlated with the fold change of the expression for the set of genes.

### BRIEF DESCRIPTION OF THE DRAWING

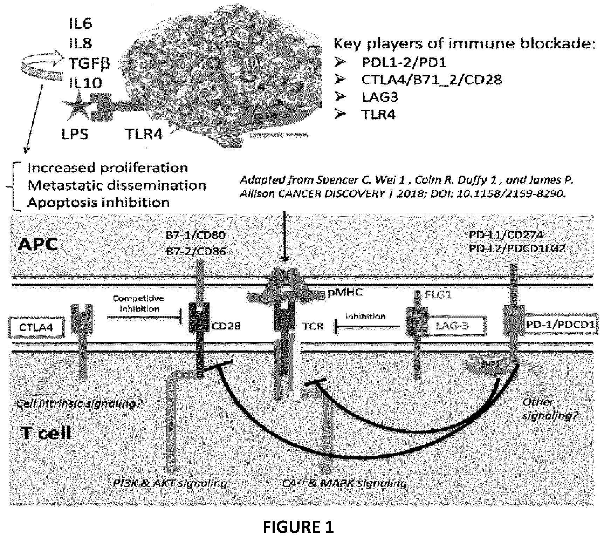

**Figure 1****: Key players of the immune blockade.** The T lymphocytes (LyT) that infiltrate the tumor (TILS) recognize the presented tumor neoantigens. The neoantigens are recognized as "non self" as they are modified proteins because of mutations. The clone of LyT that recognizes specifically the neoantigen is activated and proliferates. The recruitment of activated LyT that recognize specifically the tumor is a complex process that involves different antigen presentation mechanisms. Professional Antigen Presenting Cells (APC) present the neoantigen associated to the histocompatibility complex II (CMH2) recognized by LyT CD4+ that differentiate in LyT Helper 1 and Helper 2. Ly Th1 are key in recruitment of naïve LyT CD8+ and induce their activation. Lymphocytes T Cytotoxic (CD8+) and Natural Killer lymphocytes (NK) also recognize the neoantigen restricted to CMH1 (Histocompatibility complex 1) and are subsequently activate, and can destroy directly the tumoral cells presenting the neoantigen. The process of recruitment and activation of cytotoxic lymphocytes T CD8 is controlled by different mechanisms of negative blockade. PD-L1 and PD-L2 binds to PD-1 and directly inhibit the T receptor. CTLA-4 has a high affinity and avidity for B7-1 (CD80) an B7-2 (CD86) ligands that bind to the co-stimulatory molecule CD28. In this competitive manner, CTLA-4 blocks CD28 and has a negative blockade effect. PD-1 and CTLA-4 are highly expressed on TILs in metastatic melanoma, NSCLC, UBC, and squamous cell carcinoma of the head and neck (SCCHN). PD-1 and CTLA-4 modulate effector T cell activation, proliferation, and function through distinct, complementary mechanisms. The expression of PD-1 and CTLA-4 on tumor-infiltrating T cell populations contributes to suppression and immunological escape. *In vivo* studies have shown that tumor infiltrating lymphocytes and not peripheral T cells have been shown to be the major contributor to tumor control following anti-PD-L1 + anti-CTLA-4 antibodies therapy. LAG3 delivers inhibitory signals upon binding to ligands, such as FGL1 (responsible for LAG3 T-cell inhibitory function). Following TCR (T-cell receptor) engagement, LAG3 associates with CD3-TCR in the immunological synapse and directly inhibits T-cell activation (may inhibit antigen-specific T-cell activation in synergy with PDCD1/PD-1, possibly by acting as a co-receptor for PDCD1/PD-1, by similarity). LAG3 negatively regulates the proliferation, activation, effector function and homeostasis of both CD8(+) and CD4(+) T-cells and also mediates immune tolerance. LAG3 is constitutively expressed on a subset of regulatory T-cells (Tregs) and contributes to their suppressive function. TLR-4 activates MAPK and NF-κB pathways, implicating cell-autonomous TLR-4 signaling in regulation of carcinogenesis, in particular, through increased proliferation of tumor cells, apoptosis inhibition and metastasis. TLR-4 signaling in immune and inflammatory cells of tumor microenvironment may lead to production of pro-inflammatory cytokines (TNF, IL-1β, IL-6, IL-18, etc.), immunosuppressive cytokines (IL-10, TGF-β, etc.) and angiogenic mediators (VEGF, EGF, TGF-β, etc.).

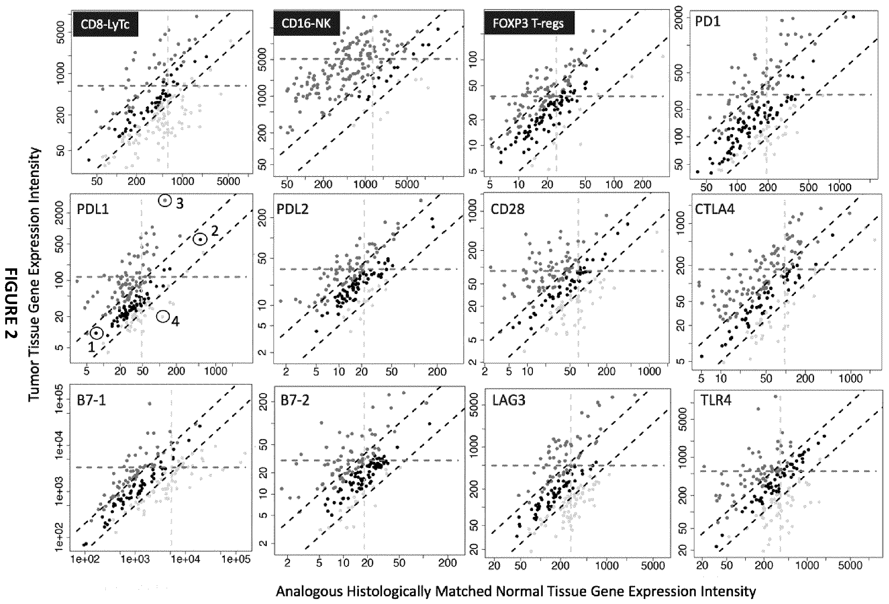

**Figure 2****:** Impact of individual variability of normal mRNA expression on assessment of key players of immune blockade mRNAs levels in tumors. Axis Y: Transcript intensity in tumors; Axis X: Transcript intensity in matched normal biopsies. Intensities are measured as relative fluorescence unit (RFU) signal as assessed with Agilent microarray technology. Each point represents a patient from Winther trial. Overexpression for a given mRNA in the tumor is denoted in red points, under-expression is denoted in green and no change is denoted in black. The 2-fold threshold (both high and low) is indicated by two dotted black lines. All 101 patients of WINTHER study with evaluable RNA data were considered. For PD-L1 gene, 4 examples are outlined: Examples 1 and 2 show no difference between Normal and Tumor tissue, but extreme variability of basal level of PD-L1 expression in normal patient. Example 3 shows high overexpression of PD-L1 in tumors. Example 4 shows under-expression in tumors. This distribution of the profiles demonstrates the necessity of using the dual biopsy tumor and normal organ matched biopsies from the same patient, as being the only way of discarding the genetic background variability of the expression level in normal tissue, and to focus only on genes relevant for tumoral transformation. The following color code will be used in all figures presenting the digital display technology:
   Red: Gene over-expressed in tumor compared to expression in normal tissue - Hypothesis: rationale for treating with treatment targeting the product of this gene.
   Black: Gene expression levels similar in tumor and normal tissues. - Hypothesis: no rationale for treating with treatment targeting the product of this gene.
   Green: Gene under-expression levels similar in tumor and normal tissues. - Hypothesis: no rationale for treatment and major risk of toxicity of the drugs targeting the product of the gene.

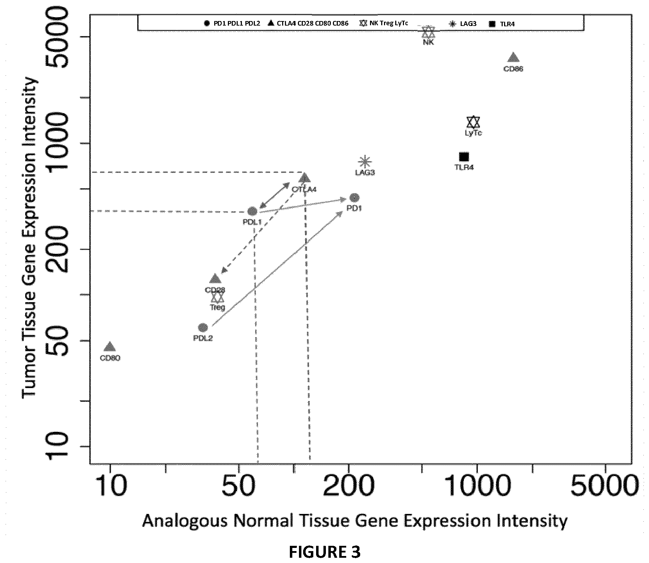

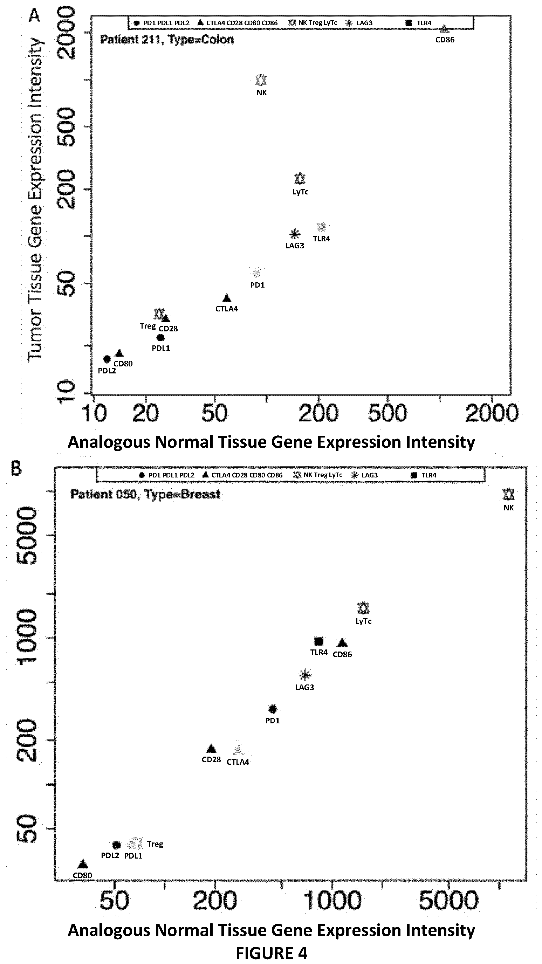

**Figures 3** **and** **4****:** The Immune Checkpoint Blockade Digital Display is a combinatorial biomarker used for simultaneous visualization of the steady state level of all key genes/mRNAs in tumor vs. normal tissue in order to assess interactions between the receptors and ligands that govern sensitivity or resistance to Immune Checkpoint Blockade treatments. Y axis: intensity of the expression in tumor tissue (using microarrays technologies or RNAseq technologies). The values are arbitrary units, but they correlate with the steady state level of the transcripts: X axis, the intensity of expression of the genes in analogous organ matched normal biopsy. For each gene transcript, the fold change is obtained by dividing the intensity in tumor with the intensity in normal biopsy.

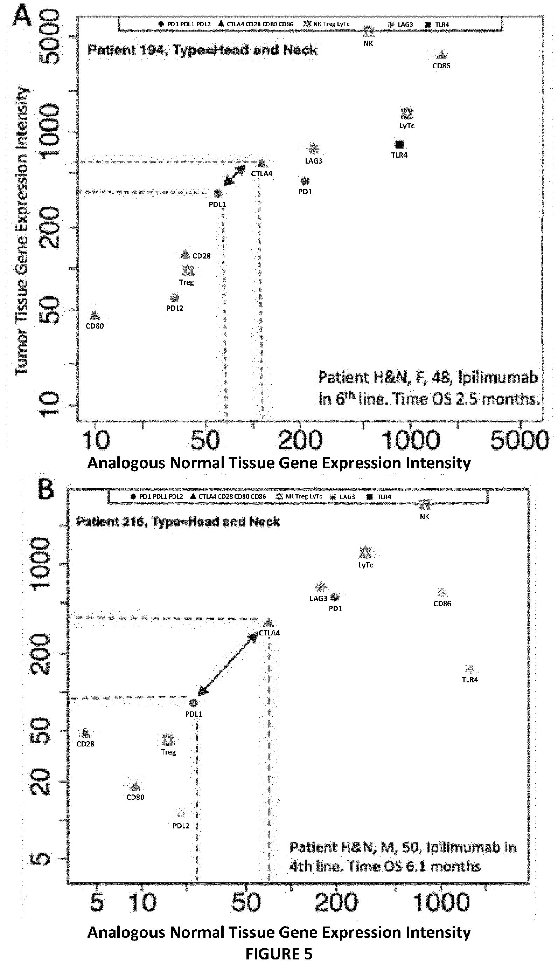

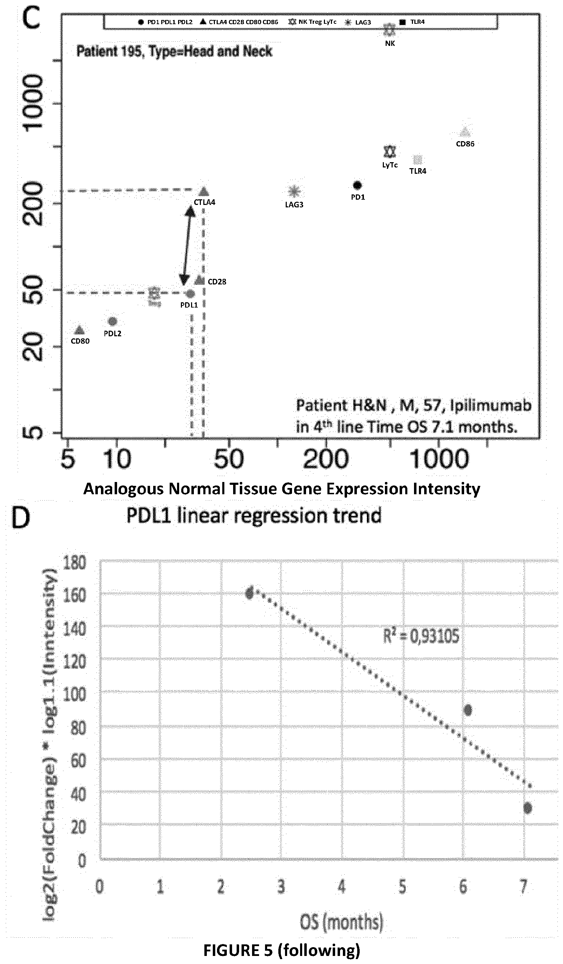

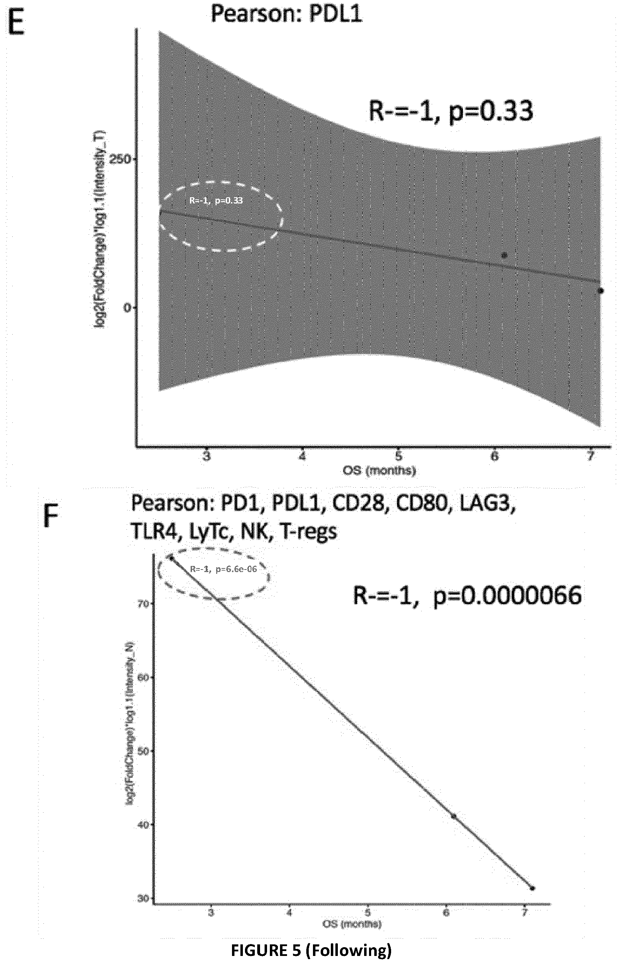

**Figure 5****:** Figures 5 A, B, C: digital display profiles of 3 patients treated with ipilimumab (anti CTLA-4): Y axis, intensity of the expression in tumors, and X axis intensity of the expression in normal matched tissue. All key partners of the immune blockade are displayed, and the fold change is interpreted as intensity of the tumor divided by intensity in the normal for each gene. Figure 5A: Intensity of CTLA-4 in tumor, reflecting the level of expression of the gene CTLA-4 is 700 RFU (relative fluorescence units), and the intensity in normal tissue is 70 RFU. Therefore, the Fold change is 10, displaying a high activation of the immune blockade on CTLA4/CD86/CD28 side. However, the immune blockade related to PDL1/PD1D is also highly activated, therefore this patient would have been an ideal candidate for a combined dual therapy associating an anti-PD-L1/PD-1 and anti-CTLA-4 therapies. Treating only with an anti-CTLA-4 may explain the poor overall survival of this patient. Trends of single gene interaction to explain variation in overall survival. Figures 5 B, C: Both immune blockades CTLA-4 and PD-L1 are highly activated, however with a different ratio than the one observed in example A. Figure 5D: Based on the observations of examples A, B and C, the following trend was identified: The highest level of PD-L1 fold change and intensity, the shortest the survival under anti-CTLA-4 treatment. This trend was confirmed by the linear regression curve, with R2 =0.93. Figure 5E: The Pearson correlation with overall survival has an interval of confidence very large, given the limited number of patients, and the Pearson correlation is not significant (pValue p=0.33). Figure 5F: combinatorial interactions: For each of the correlations, all possible combinations by 4, 5, 6, 7, 8, 9 and 10 genes among all the key players were tested and ranked by order of significance. The top correlators were selected. The best correlator with the overall survival of patients treated with ipilumumab is provided by the interaction of 8 different genes (key players of immune blockade and specific markers of tumor infiltrating immune cells: PD-1, PD-L1, CD28, CD80, CD8, LAG3, TLR4, CD16 and FOXP3. The interaction of the genes of the best correlator is the mean of fold change multiplied by intensity in tumor, for each of the 8 genes. The Pearson correlation demonstrates a negative correlation R=-1 with and a very high significance (p value p= 0.0000066).

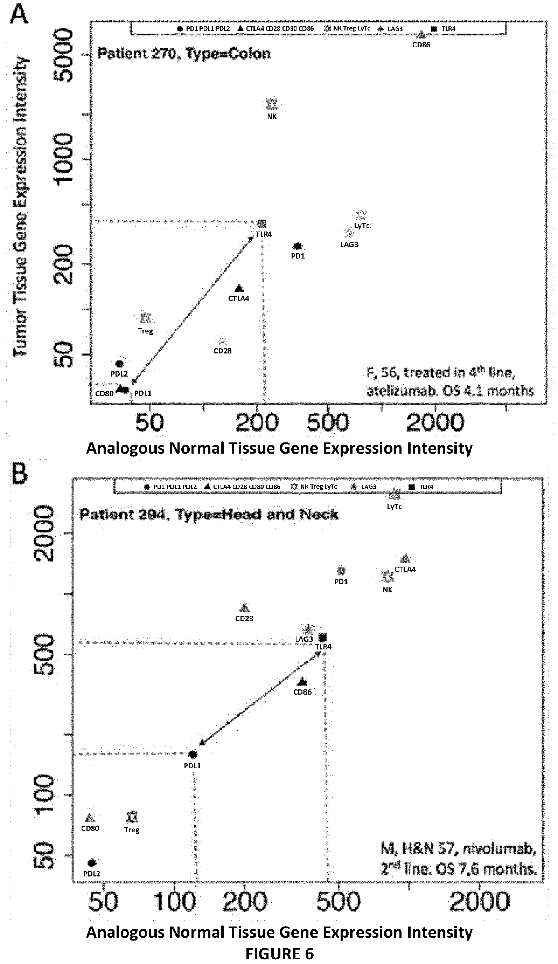

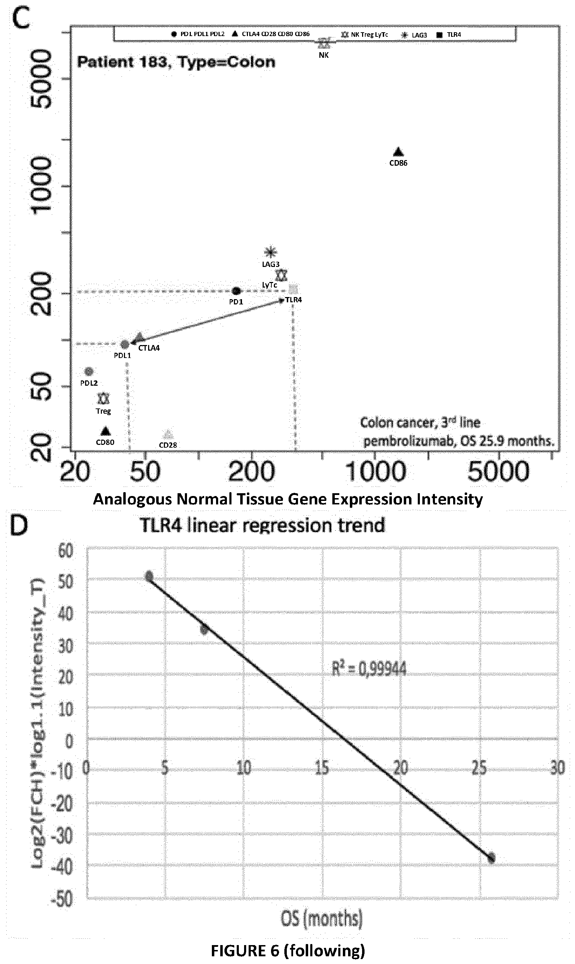

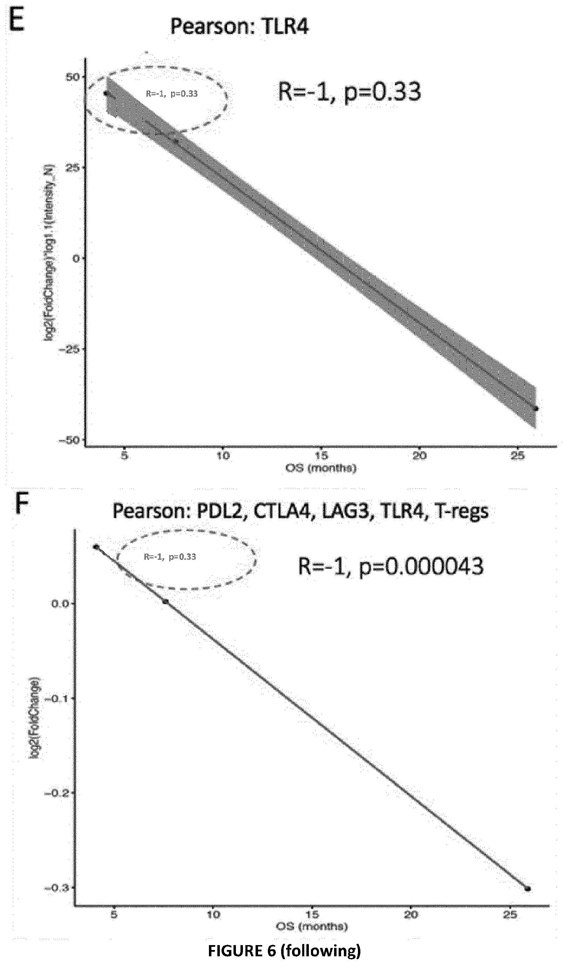

**Figure 6****:** Figure 6 A, B, C: Digital display profiles of 3 patients treated with anti PD-L1/PD-1 antibodies. Y axis, intensity of the expression in tumors, and X axis intensity of the expression in normal matched tissue. All key partners of the immune blockade are displayed, and the fold change is interpreted as intensity of the tumor divided by intensity in the normal for each gene. Figure 6D: Trend of single gene interaction to explain variation in overall survival. The highest level of TLR4 fold change and intensity, the shortest the survival. However, this is only a trend, as the interval of confidence is very large, given the limited number of patients. Figure 6E: The Pearson correlation of TLR4 with overall survival confirm the trend, but is not significant (p=0.33). Figure 6F: Combinatorial interactions: For each of the correlations with overall survival, all possible combinations by 4, 5, 6, 7, 8, 9 and 10 genes among all the key players were tested and ranked by order of significance. The top correlators were selected. The best correlator is provided by the interaction of 5 different genes (key players of immune blockade and specific markers of tumor infiltrating immune cells): PDL2, CTLA4, LAG3, TLR4 and FOXP3. The interaction of the genes of the best correlator is the mean of fold changes for each of the 5 genes. The Pearson correlation demonstrates a negative correlation R=-1 with and a very high significance (p value p= 0.000043).

### DETAILED DESCRIPTION OF THE INVENTION

The inventors provide a new method for generating information allowing to analyze simultaneously and globally the key players of the immune-negative blockade. More specifically, the method allows to visualize simultaneously, for each individual patient, the steady state of all key genes (e.g., Figure 1 and Table 1) by comparing their expression in a tumor sample and a normal sample from the same individual patient in order to assess interaction between receptors and ligands that govern lymphocyte T negative blockade. In addition, the method enables to monitor the level of infiltration of the tumor by specific types of immune cells, especially by analyzing the differential expression between the tumor and normal tissue from the same patient of specific markers of immune cells.

Then, the susceptibility to have a therapeutic benefit of a treatment with an immune checkpoint blockade therapy for a particular subject suffering from a cancer can be assessed based on the expression of the genes encoding the key players in the tumor and a normal histologically matched sample from the same subject and their expression intensity in the tumor sample.

Accordingly, the present invention relates to a method for determining if a subject suffering from a cancer is susceptible to have a therapeutic benefit of a treatment with an immune checkpoint blockade therapy, comprising
- providing mRNA expression level in a tumor sample and a normal histologically matched sample of a set of genes comprising genes of one or more of the following groups of genes: 1) PD-1, PD-L1, and PD-L2; 2) CTLA-4, CD80, CD86, and CD28; 3) LAG3; 4) TLR-4 and 5) CD8, CD16 and FOXP3; the set of genes comprising at least the genes of the group 1) or 2); and wherein the tumor and normal histologically matched samples are from the same subject;
- classifying the genes into three classes: i) a first class in which genes are overexpressed in the tumor sample in comparison to the normal histologically matched sample; ii) a second class in which gene are expressed at a similar level in the tumor sample in comparison to the normal histologically matched sample; and iii) a third class in which genes are underexpressed in the tumor sample in comparison to the normal histologically matched sample; and
- displaying the genes classified into the three classes.

Then, in a first step, the inventors have selected the key players to be taken into consideration in the method of the present invention.

In a first group of genes, they included the genes useful for assessing the responsiveness of a patient to an immune checkpoint blockade therapy, in particular a therapy targeting PD-1/PD-L1. This set of genes includes PD-1, PD-L1 and PD-L2. The role of PD-1, PD-L1 and PD-L2 is well-known. PD-1 negatively regulates T cell activation through interaction with PD-L1 and PD-L2. The therapy targeting PD-1/PD-L1 aims to block the interaction between PD-1 and PD-L1 and/or PD-L2 so as to remove this blockade.

In a second group of genes, they included the genes useful for assessing the responsiveness of a patient to an immune checkpoint blockade therapy, especially a therapy targeting CTLA-4. This set of genes includes CTLA-4, CD80, CD86, and CD28. Briefly, CTLA-4 is immediately upregulated following TCR engagement. CTLA-4 negatively regulates TCR signaling through competition with the costimulating CD28 for the binding of CD80/CD86 for which CTLA-4 has a higher affinity and avidity. The therapy targeting CTLA-4 aims to block the interaction between CTLA-4 and CD80 or CD86 so as to remove this blockade.

As CTLA-4 and PD-1 act at least in part through a similar molecular mechanism of attenuating CD28-mediated costimulation, it seems important to provide in the method information about the expression of genes relating to PD-1 and those relating to CTLA-4 (i.e., groups 1 and 2) in order to provide a global assessment of the immune-negative blockade.

LAG3 is an inhibitory receptor on antigen activated T-cells. It delivers inhibitory signals upon binding to ligands, such as FGL1. Following TCR engagement, LAG3 associates with CD3-TCR in the immunological synapse and directly inhibits T-cell activation. A synergistic effect with PD-1 has been mentioned. LAG3 may act as a coreceptor of PD-1. Then, it could be interesting to provide data on the expression of LAG3. Accordingly, a third group comprises LAG3 and optionally its main ligand FGL1.

The stimulation of TLR4, in particular its overexpression, has also an important role in the stimulation of the immune response. Therefore, the fourth group comprises TLR4.

Finally, as discussed above, the inventors consider that the presence of specific immune cells in the tumor is also a key aspect that needs to be taken into consideration. The specific immune cells can be the cytotoxic CD8+ T lymphocytes, the NK cells and/or T T More specifically, the selected specific markers are the following:
1. Level of infiltration of the tumor by Cytotoxic Lymphocytes T CD8 (LyTc). The specific marker of these cells is CD8.
2. Level of the infiltration of the tumor by Natural Killers cells (NK). The specific marker of these cells is CD16; and
3. Level of the infiltration of the tumor by a specific population of Lymphocytes T called T regulatory (T-regs). The specific marker of these cells is FOXP3.

More particularly, it can be CD8A or CD8B. Indeed, the process of immune response starts with activation of the T receptor (TCR) of CD8 cytotoxic lymphocytes. CD8 lymphocyte TCR activated by recognizing neo-antigens from the tumoral cells are recruited and, following the activation, the clone is expanded, proliferate and activated, leading to the antitumor activity in cooperation with NK cells and T-reg cells. However, the activation is controlled by a complex negative blockade mechanism. The presence of activated cytotoxic CD8+ T lymphocytes is necessary for having the antitumor activity when the negative blockade is removed by the immune checkpoint blockade therapy.

Then, a fifth group comprises a marker specific of CD8+ T lymphocytes, a marker specific of NK cells and a marker specific of T reg cells, preferably CD8, CD16 and FOXP3, more preferably CD8A, CD16 and FOXP3.

Additional genes could be taken into consideration in the present method. For instance, TIM3 (T cell membrane protein-3 (Uniprot ID Q8TDQ0), TIGIT and its ligands (e.g., CD113), CD96 and its ligands (e.g., CD111), VISTA, ICOS, 0X40, GITR or 4-IBB can be added in the set of genes to be studied for their expression.

**Table 1 - Table with the description of the key players.**

| Name | Official names and aliases | UniProt ID | GeneCard ID |
|---|---|---|---|
| PD-1 | Programmed cell death 1, PDCD1, CD279, PD-1, PD1, SLEB2, hPD-1, hPD-I, hSLE1, | Q15116 | PDCD1 |
| PD-L1 | Programmed death-ligand 1, CD274, B7-H, B7H1, PDCD1L1, PDCD1LG1, PDL1, CD274 molecule | Q9NZQ7 | CD274 |
| PD-L2 | Programmed cell death 1 ligand 2, PDCD1LG2, B7DC, Btdc, CD273, PD-L2, PDCD1L2, PDL2, bA574F11.2 | Q9BQ51 | PDCD1LG2 |
| CTLA-4 | Cytotoxic T-lymphocyte-associated protein 4, CTLA4, ALPS5, CD, CD152, CELIAC3, CTLA-4, GRD4, GSE, IDDM12 | P16410 | CTLA4 |
| CD80 | T-lymphocyte activation antigen CD80, Cluster of differentiation 80, CD80, B7, B7-1, B7.1, BB1, CD28LG, CD28LG1, LAB7, CD80 molecule | P33681 | CD80 |
| CD86 | T-lymphocyte activation antigen CD86, Cluster of Differentiation 86, CD86, B7-2, B7.2, B70, CD28LG2, LAB72, CD86 molecule | CD86 | P42081 |
| CD28 | T-cell-specific surface glycoprotein CD28, Cluster of Differentiation 28, CD28, Tp44, CD28 molecule | P10747 | CD28 |
| LAG3 | Lymphocyte activation gene 3 protein, CD223, | P18627 | LAG3 |
| TLR4 | Toll-like receptor 4, TLR4, ARMD10, CD284, TLR-4, TOLL | O00206 | TLR4 |
| CD8 | T-cell surface glycoprotein CD8 alpha chain, Cluster of Differentiation 8a, CD8A, CD8, Leu2, MAL, p32, CD8a molecule | P01732 | CD8A |
| CD16 | CD16a Antigen, FCGR3, CD16A, IGFR3, FCG3, CD16 | P08637 | FCGR3A |
| FOXP3 | Forkhead Box P3, FOXP3delta7, DIETER, AIID, PIDX, XPID, JM2 | Q9BZS1 | FOXP3 |
| CD8B | T-cell surface glycoprotein CD8 beta chain, CD8B | P10966 | CD8B |

Then, the method takes into consideration a set of genes comprising genes of one or more of the following groups of genes: group 1) PD-1, PD-L1, and PD-L2; group 2) CTLA-4, CD80, CD86, and CD28; group 3) LAG3; group 4) TLR4 and group 5) CD8, CD16 and FOXP3; the set of genes comprising at least the genes of the group 1) or 2).

In one aspect, the method takes into consideration a set of genes comprising the following genes: PD-1, PD-L1, and PD-L2; and/or CTLA-4, CD80, CD86, and CD28. In a preferred aspect, the method takes into consideration a set of genes comprising the following genes: PD-1, PD-L1, PD-L2, CTLA-4, CD80, CD86, and CD28.

Optionally, the set of genes is selected in one of the following sets: a) PD-1, PD-L1, PD-L2, CTLA-4, CD80, CD86, CD28, LAG3, TLR4, CD8, CD16 and FOXP3; b) PD-1, PD-L1, PD-L2, CTLA-4, CD80, CD86, LAG3 and TLR4; c) PD-1, PD-L1, PD-L2, CTLA-4, CD80, CD28, CD8, CD16 and FOXP3; d) PD-1, PD-L1, PD-L2, LAG3, TLR4, CD8, CD16 and FOXP3; and e) CTLA-4, CD80, CD86, CD28, LAG3, TLR4, CD8, CD16 and FOXP3.

In a preferred embodiment, the set of genes comprises, essentially consists in or consists in the following genes PD-1, PD-L1, PD-L2, CTLA-4, CD80, CD86, CD28, LAG3, TLR4, CD8, CD16 and FOXP3.

Optionally, in particular as detailed above, the set of genes may further comprise additional genes. However, in a preferred aspect, the total number of genes in the set of genes is no more than 15, 14, 13, 12, or 11 genes.

The mRNA expression level is determined for the genes of the set in a tumor sample and in a normal histologically matched sample from the same subject or patient. Optionally, the method may comprise a preliminary step of providing a tumor sample and in a normal histologically matched sample from the same subject or patient.

The subject or patient suffers from a cancer or has a cancer. In particular, the cancers or tumors more particularly considered in the present invention are lung cancer, especially NSCLC (non-small cell lung cancer), breast cancer (in particular the triple negative breast cancer), colorectal cancers, kidney cancer, melanomas, rhabdomyosarcomas, brain cancers, liver cancers, head and neck cancers, stomach cancers, ovary cancers, pancreatic cancers, liposarcomas and other types of solid tumors.

Then, two samples are necessary, namely one tumor sample and one normal sample from the same patient. Preferably, the tumour sample and the normal sample provides from the same type of tissue. More particularly, the tumor and normal samples are histologically matched tissues. Tumor tissue is a fragment obtained from the tumor or metastatic lesions, (usually provided in interventional radiology) and containing at least 50% tumoral cells, immune infiltrating cells, stromal cells, vessels. The normal tissue is a fragment from histologically matched normal tissue (usually provided in fibroscopy or endoscopy units) and containing at least 30% normal cells (e.g., epithelial cells). DNA and total RNA preparations are performed and only high quality nucleic acids quality are used for transcriptomics investigations (measure of differential expression of mRNA and optionally miRNA between the tumor and normal tissues.

Typically, the samples can be provided by biopsies. Non-exhaustively, examples of pairs of tumor with corresponding histological normal reference tissue are the followings:
1. lung cancer adenocarcinomas or derived metastases - bronchial normal mucosa
2. breast cancer tumors or derived metastases - normal epithelial breast cells
3. colon cancers adenocarcinomas or derived metastases - normal colon mucosa
4. kidney cancers or derived metastases - normal kidney cells
5. melanomas or derived metastases - synchronous naevi
6. rhabdomyosarcomas or derived metastases - normal muscle tissue
7. liver carcinomas or derived metastases - normal liver cells
8. Oral-pharyngeals or head and neck tumors (ORL) - normal buccal mucosa
9. Stomach carcinomas or derived metastases - normal stomach mucosa
10. Ovary cancer - normal Fallope tube mucosa
11. pancreatic cancers - normal parenchimatous tissue from pancreas
In order to optimize the tumor characterization, the inventors selected parameters that have to be analysed in order to establish the status of the intervention points that can be targeted by a class of drugs.

The expression levels are determined by measuring mRNA level. The determination of the expression level variation for these mRNA is carried out by comparing the expression levels in a tumor tissue and in the corresponding normal tissue. Technologies that can be used are well known in the art and comprise Northern analysis, mRNA or cDNA microarrays, RT-PCT (in particular quantitative RT-PCR) and the like. Alternatively, the level of expression can be determined with a ship comprising a set of primers or probes specific for the set of genes. Expression levels obtained from cancer and normal samples may be normalized by using expression levels of proteins which are known to have stable expression such as RPLPO (acidic ribosomal phosphoprotein PO), TBP (TATA box binding protein), GAPDH (glyceraldehyde 3-phosphate dehydrogenase) or β-actin.

Then, based on the mRNA expression levels of the genes of the set, the genes are classified into three classes: i) a first class in which genes are overexpressed in the tumor sample in comparison to the normal histologically matched sample; ii) a second class in which gene are expressed at a similar level in the tumor sample in comparison to the normal histologically matched sample; and iii) a third class in which genes are underexpressed in the tumor sample in comparison to the normal histologically matched sample.

The classification is based on the expression fold change in the tumor sample in comparison to the normal histologically matched sample.

In a preferred aspect, a gene is overexpressed when the fold change between the tumor sample and the normal histologically matched sample is higher than 1.3, a gene is expressed at a similar level when the fold change is between -1.3 and 1.3, and a gene is underexpressed when the fold change is lower than -1.3. However, different threshold of fold change may also be used, for instance a first class with a fold change higher than x, a second class with a fold change is between -x and x, and a third class with a fold change lower than -x, x being a number between 1 and 5, preferably between 1 and 4, between 1 and 3 or between 1 and 2. For instance, x could be 1, 1.1, 1.2, 1.3, 1.4, 1.5, 1.6, 1.7, 1.8, 1.9 or 2.

The method may consider a more precise classification including more classes. For instance, the first class may be subdivided into a class with weak overexpression and another with high overexpression. Similarly, the third class may be subdivided into a class with weak underexpression and another with high underexpression.

Optionally, the mRNA fold change of a gene can be corrected by considering the expression of the miRNA of the gene in order to adjust possible miRNA intervention in translation.

More preferably, a mean miRNAs fold change for each gene is calculated as the average of the miRNA fold changes between the tumor sample and the normal histologically matched sample for the gene. Then, a corrected mRNA fold change is calculated by dividing the mRNA fold change between the tumor sample and the normal histologically matched sample of the gene (mRNA T*v*N fold change) by the mean fold change for the miRNAs of the gene (mean miRNA T*v*N fold change), and the corrected mRNA fold change of the gene is then used in the method for classifying the genes into the three classes. Levels of miRNAs for the genes are determined in the tumor and normal samples. The miRNAs most likely to be involved in the gene expression regulation can be determined by using Target scan {www.targetscan.org/}. For instance, the top 5 miRNAs can be selected for each gene. Table 2 provides a list of the top 5 miRNAs for the genes. Accordingly, the levels of 5 miRNAs for each gene can be determined in the tumor sample and the normal histologically matched sample. The method for measuring miRNA are well-known in the art. Then, a fold change Tumor versus Normal tissue is determined for the 5 miRNAs and a mean fold change for each gene is calculated as the average of the fold changes of the 5 miRNAs.

**Table 2 - Top 5 miRNA for each gene. (From TARGETSCAN database)**

| **Gene** | **Gene Official Name** | **miRNA1** | **miRNA1** | **miRNA3** | **miRNA4** | **miRNA5** |
|---|---|---|---|---|---|---|
| PD1 | PDCD1 | hsa-miR-195-5p | hsa-miR-16-5p | hsa-miR-424-5p | hsa-miR-497-5p | hsa-miR-15b-5p |
| PDL1 | CD274 | hsa-miR-17-5p | hsa-miR-106a-5p | hsa-miR-519d-3p | hsa-miR-20b-5p | hsa-m iR-93-5p |
| PDL2 | PDCD1LG2 | hsa-miR-20b-5p | hsa-miR-17-5p | hsa-miR-106a-5p | hsa-miR-20a-5p | hsa-miR-20b-5p |
| CTLA4 | CTLA4 | hsa-miR-155-5p | hsa-miR-496.1 | hsa-miR-142-3p.2 | hsa-miR-142-3p.2 | hsa-m iR-6803-3p |
| CD80 | CD80 | hsa-miR-455-3p.1 | hsa-miR-146b-5p | hsa-miR-146a-5p | hsa-miR-7153-5p | hsa-miR-4681 |
| CD86 | CD86 | hsa-let-7i-5p | hsa-let-7e-5p | hsa-let-7c-5p | hsa-let-7f-5p | hsa-miR-4500 |
| CD28 | CD28 | hsa-miR-424-5p | hsa-miR-497-5p | hsa-miR-15a-5p | hsa-miR-6838-5p | hsa-miR-195-5p |
| LyTc | CD8A | hsa-miR-326 | hsa-miR-330-5p | hsa-miR-5591-3p | hsa-miR-660-5p | hsa-miR-7973 |
| TLR4 | TLR4 | hsa-miR-140-5p | hsa-miR-542-3p | hsa-miR-489-3p | hsa-miR-5195-3p | hsa-miR-145-5p |
| NK | FCGR3A | hsa-miR-6893-3p | hsa-miR-370-3p | hsa-miR-326 | hsa-miR-330-5p | hsa-miR-7153-3p |
| Treg | FOXP3 | hsa-miR-6885-5p | hsa-miR-939-5p | hsa-miR-3196 | hsa-miR-3191-3p | hsa-miR-1343-5p |
| LAG3 | LAG3 | hsa-miR-6829-5p | hsa-mi R-4515 | hsa-miR-1269b | hsa-miR-1269a | hsa-miR-3692-5p |

The steps of fold change calculation and classification can be computer-implemented steps.

Another information that will be used in the method of the present invention are the intensity of the mRNA expression in tumour and in histological matched normal tissue from the same patients. The intensity can be assessed by measuring the signal that can be detected using of the microarrays technologies that enable to assess the Relative Fluorescent Units, whose value correlates with the steady state level of the mRNA (or microRNA). Detection can be performed also by RNAseq technologies (such as Next generation sequencing) and the intensities are assessed by the counts of the number of reads (tag), which also correlates with the steady state levels of the mRNA studies. Globally, technologies used enable to identify and measure the intensities/expression levels of all the types of mRNA (miRNA). Several technologies are exemplified, Agilent Microarrays, Affymetrix microarrays, Illumina RNAseq, and many others, including but not limited to RT-QPCR, Nanostring etc. The intensities measured in tumour tissues divided by the intensities measured in Normal tissues generates the Fold change of mRNAs and miRNAs.

Then, the method comprises a step of displaying the genes of the set classified into the three classes. This step of display is preferably a computer-implemented step.

In a particular aspect, the genes classified into the three classes are displayed as a graph, especially a point chart, each point representing the expression level of one gene of the set. In a preferred aspect, the graph shows the expression intensity of the genes of the set in the tumor sample on the ordinate and the expression intensity in the normal histologically matched sample on the abscissa.

Preferably, each point is associated with the name of the gene.

In a preferred aspect, each of the three classes is displayed with a distinct mode allowing to differentiate the first class from the second and third classes. In a particular aspect, each of the three classes is displayed with a distinct mode allowing to differentiate the first class, the second class and third class from each other's. The distinct mode can be a different color (e.g., red, black and green points) or a different form (e.g., circle, square and triangle).

Optionally, the display can make apparent the different group of genes, e.g., groups 1, 2, 3, 4 and/or 5. For instance, a color can be associated to each class and a form to each group.

The main goal of the display is to make available which gene is in which class and the intensity of expression in the tumor sample for the set of genes.

Based on this display, the person skilled in art has at his/her disposal the information allowing to determining if a subject suffering from a cancer is susceptible to have a therapeutic benefit of a treatment with an immune checkpoint blockade therapy or not. Indeed, this susceptibility is based on the global information provided by the display

The susceptibility to have a therapeutic benefit of a treatment with an immune checkpoint blockade therapy is assessed based on the presence of the genes of groups in one of the three classes and the expression intensity of the genes of groups in the tumor sample. The determination of the susceptibility can be part of the method of the present invention.

The method may further comprise a step of selecting a patient susceptible to have a therapeutic benefit of a treatment with an immune checkpoint blockade therapy. It can also comprise a step of administering a therapeutic amount of the immune checkpoint blockade therapy to the selected patient.

The method may also or alternatively comprise a step of selecting a patient who is not susceptible to have a therapeutic benefit of a treatment with an immune checkpoint blockade therapy or is a non-responder. Then, the selected patient will not be suitable to receive a therapeutic benefit of a treatment with an immune checkpoint blockade therapy because he/she would be a non-responder or because the treatment will likely be associated with adverse side effects.

Generally, the more of genes from the set belonging to the first class and the higher is the expression intensity, the higher is the likelihood that the patient will have a therapeutic benefit of a treatment with an immune checkpoint blockade therapy. On the opposite, the presence of genes of the set in the third class would be indicative of the lack of response and/or the occurrence of adverse side effects.

Within the context of this invention, "responder", "responsive" or "have a therapeutic benefit" refers to a patient who responds to a treatment of cancer, i.e. the volume of the tumor is decreased, at least one of his symptoms is alleviated, or the development of the cancer is stopped, or slowed down. Typically, a subject who responds to a cancer treatment is a subject who will be completely treated (cured), i.e., a subject who will survive to the cancer. A subject who responds to a cancer treatment is also, in the sense of the present invention, a subject who have an overall survival higher than the mean overall survival known for the particular cancer. By "good responder" or "susceptible to have a therapeutic benefit" is intended a patient who shows a good therapeutic benefit of the treatment, that is to say a longer disease-free survival, a longer overall survival, a decreased metastasis occurrence, a decreased tumor growth and/or a tumor regression in comparison to a population of patients suffering from the same cancer and having the same treatment.

Within the context of this invention, "non-responder" refers to a subject who does not respond to a treatment of cancer, i.e. the volume of the tumor does not substantially decrease, or the symptoms of the cancer in the subject are not alleviated, or the cancer progresses, for example the volume of the tumor increases and/or the tumor generates local or distant metastasis. The terms "non-responder" also refer to a subject who will die from the cancer, or will have an overall survival lower than the mean overall survival known for the particular cancer. By "poor responder" or "non-responder" is intended a patient who shows a weak therapeutic benefit of the treatment, that is to say a shorter disease-free survival, a shorter overall survival, an increased metastasis occurrence and/or an increased tumor growth in comparison to a population of patients suffering from the same cancer and having the same treatment.

In a particular aspect, when some genes of the group 1) are ranked in the first class (e.g., at least two genes of the group 1), in particular at least PD-1 and PD-L1) and optionally their expression intensity in the tumor sample is high, then the subject is susceptible to have a therapeutic benefit of a treatment with an immune checkpoint blockade therapy, in particular an immune checkpoint blockade therapy targeting PD-1/PD-L1. Optionally, the genes of the group 1) are all ranked in the first class (i.e., PD-1, PD-L1 and PD-L2) and their expression intensity in the tumor sample is high.

On the opposite, when some genes of the group 1) are ranked in the second or third class (e.g., in particular at least PD-1 or PD-L1), then the subject has a low susceptibility to have a therapeutic benefit of a treatment with an immune checkpoint blockade therapy, in particular an immune checkpoint blockade therapy targeting PD-1/PD-L1, or the subject is a non-responder. In other word, the patient has a high likelihood to be non-responder or that adverse side effect occurs. This low susceptibility to have a therapeutic benefit of a treatment with an immune checkpoint blockade therapy could be even lower when their expression intensity in the tumor sample is low. Optionally, the genes of the group 1) are all ranked in the second or third class (i.e., PD-1, PD-L1 and PD-L2).

In another particular aspect, when some genes of the group 2) are ranked in the first class (e.g., at least two genes of the group 2), in particular at least CTLA-4 and CD28) and optionally their expression intensity in the tumor sample is high, then the subject is susceptible to have a therapeutic benefit of a treatment with an immune checkpoint blockade therapy, in particular an immune checkpoint blockade therapy targeting CTLA-4. Optionally, at least 2, 3 or all genes of the group 2) are ranked in the first class and their expression intensity in the tumor sample is high. For instance, the genes ranked in the first class could be CTLA-4 and CD28; CTLA-4 and; CTLA-4 and CD86; CTLA-4, CD80 and CD86; CTLA-4, CD28 and CD86; CTLA-4, CD80 and CD28; or CTLA-4, CD80, CD86 and CD28.

Optionally, when some genes of the group 2) are ranked in the first class (e.g., at least two genes of the group 2), in particular at least CTLA-4 and CD28) and optionally their expression intensity in the tumor sample is low, then the subject has a low susceptibility to have a therapeutic benefit of a treatment with an immune checkpoint blockade therapy, in particular an immune checkpoint blockade therapy targeting CTLA-4, or the subject is a non-responder. Optionally, at least the expression intensity of CTLA-4 or CD28 is low in the tumor sample. Optionally, at least the expression intensity of CTLA-4 and CD28; CTLA-4 and; CTLA-4 and CD86; CTLA-4, CD80 and CD86; CTLA-4, CD28 and CD86; CTLA-4, CD80 and CD28; or CTLA-4, CD80, CD86 and CD28, is low in the tumor sample.

On the opposite, when some genes of the group 2) are ranked in the second or third class (e.g., at least two genes of the group 2), in particular at least CTLA-4 and CD28), then the subject has a low susceptibility to have a therapeutic benefit of a treatment with an immune checkpoint blockade therapy, in particular an immune checkpoint blockade therapy targeting CTLA-4, or the subject is a non-responder. In other word, the patient has a high likelihood to be non-responder or that adverse side effect occurs. This low susceptibility to have a therapeutic benefit of a treatment with an immune checkpoint blockade therapy could be even lower when their expression intensity in the tumor sample is low. Optionally, at least 2, 3 or all genes of the group 2) are ranked in the second or third class. For instance, the genes ranked in the first class could be CTLA-4 and CD28; CTLA-4 and; CTLA-4 and CD86; CTLA-4, CD80 and CD86; CTLA-4, CD28 and CD86; CTLA-4, CD80 and CD28; or CTLA-4, CD80, CD86 and CD28.

In a third particular aspect, when some genes of the group 1) are ranked in the first class (e.g., at least two genes of the group 1), in particular at least PD-1 and PD-L1) and optionally their expression intensity in the tumor sample is high; and when some genes of the group 2) are ranked in the first class (e.g., at least two genes of the group 2), in particular at least CTLA-4 and CD28) and optionally their expression intensity in the tumor sample is high; then the subject is susceptible to have a therapeutic benefit of a treatment with a combined immune checkpoint blockade therapy targeting PD-1/PD-L1 and CTLA-4. Optionally, the genes of the group 1) are all ranked in the first class (i.e., PD-1, PD-L1 and PD-L2). Optionally, at least 2, 3 or all genes of the group 2) are ranked in the first class. For instance, the genes ranked in the first class could be CTLA-4 and CD28; CTLA-4 and; CTLA-4 and CD86; CTLA-4, CD80 and CD86; CTLA-4, CD28 and CD86; CTLA-4, CD80 and CD28; or CTLA-4, CD80, CD86 and CD28.

In a fourth particular aspect, when some genes of the group 3) are ranked in the first class (e.g., at least the gene LAG3) and optionally their expression intensity in the tumor sample is high, then the subject is susceptible to have a therapeutic benefit of a treatment with an immune checkpoint blockade therapy, in particular an immune checkpoint blockade therapy targeting LAG3.

On the opposite, when some genes of the group 3) are ranked in the second or third class (e.g., at least the gene LAG3), then the subject has a low susceptibility to have a therapeutic benefit of a treatment with an immune checkpoint blockade therapy, in particular an immune checkpoint blockade therapy targeting LAG3, or the subject is a non-responder. In other word, the patient has a high likelihood to be non-responder or that adverse side effect occurs. This low susceptibility to have a therapeutic benefit of a treatment with an immune checkpoint blockade therapy could be even lower when their expression intensity in the tumor sample is low. Optionally, the genes of the group 3) are all ranked in the second or third class (i.e., LAG3 and FLG1).

The genes of the groups 4) and 5) are additional elements in order to predict the susceptible to have a therapeutic benefit of a treatment with an immune checkpoint blockade therapy. In other words, they could be used in order to refine the prediction based on the groups 1) and 2). Alternatively, the prediction can also be based on the groups 4) and 5).

Accordingly, when some genes of the group 4) and 5) are ranked in the first class (e.g., TLR4, CD8, CD16 and/or FOXP3), and optionally their expression intensity in the tumor sample is high, then this is indicative of the subject susceptible to have a therapeutic benefit of a treatment with an immune checkpoint blockade therapy. On the opposite, wherein when some genes of the group 4) and 5) are ranked in the second or third class (e.g., TLR4, CD8, CD16 and FOXP3), then this is indicative of the subject having low susceptibility to have a therapeutic benefit of a treatment with an immune checkpoint blockade therapy or who is a non-responder.

In a particular aspect, when CD8, CD16 and/or FOXP3 is ranked in the first class, and optionally its expression intensity in the tumor sample is high, then this is indicative of the subject susceptible to have a therapeutic benefit of a treatment with an immune checkpoint blockade therapy. On the opposite, wherein when CD8, CD16 and/or FOXP3 is ranked in the second or third class, then this is indicative of the subject having low susceptibility to have a therapeutic benefit of a treatment with an immune checkpoint blockade therapy or who is a non-responder.

In another particular aspect, when TLR4 is ranked in the first class, and optionally its expression intensity in the tumor sample is high, then this is indicative of the subject susceptible to have a therapeutic benefit of a treatment with an immune checkpoint blockade therapy. On the opposite, wherein when TLR4 is ranked in the second or third class, then this is indicative of the subject having low susceptibility to have a therapeutic benefit of a treatment with an immune checkpoint blockade therapy or who is a non-responder.

The immune checkpoint blockade therapies are well-known in the art. In particular, it could be an immune checkpoint blockade therapy targeting PD-1/PD-L1. Alternatively, it could be an immune checkpoint blockade therapy targeting CTLA-4. It could also be an immune checkpoint blockade therapy targeting both PD-1/PD-L1 and CTLA-4.

An immune checkpoint blockade therapy targeting PD-1/PD-L1 can be for instance an inhibiting anti-PD-1 antibody, an inhibiting anti-PD-Ll antibody or an inhibiting anti-PD-L2 antibody, preferably an inhibiting anti-PD-1 antibody or an inhibiting anti-PD-Ll antibody. Such antibodies are well-known in the art. Several antibodies have already been accepted as drug. Others are still in clinical development.

The inhibiting anti-PD-1 antibody can be for instance selected from PDR001 (Novartis), Nivolumab (Bristol-Myers Squibb), Pembrolizumab (Merck & Co), Pidilizumab (CureTech), MED10680 (Medimmune), REGN2810 (Regeneron), TSR-042 (Tesaro), PF-06801591 (Pfizer), BGB-A317 (Beigene), BGB-108 (Beigene), INCSHR1210 (Incyte), or AMP-224 (Amplimmune).

The inhibiting anti-PD-Ll antibody can be for instance selected from FAZ053 (Novartis), Atezolizumab (Genentech/Roche), Avelumab (Merck Serono and Pfizer), Durvalumab (Medlmmune/AstraZeneca), or BMS-936559 (Bristol-Myers Squibb).

An immune checkpoint blockade therapy targeting CTLA-4 can be for instance an inhibiting anti-CTLA-4 antibody. Such antibodies are well-known in the art. Several antibodies have already been accepted as drug. Others are still in clinical development. For instance, the CTLA-4 inhibitor can be selected from ipilimumab, tremelimumab, and AGEN-1884.

An immune checkpoint blockade therapy targeting LAG3 can be for instance an inhibiting anti-LAG3 antibody. Such antibodies are well-known in the art. Several antibodies have already been accepted as drug. Others are still in clinical development. For instance, the LAG-3 inhibitor can be selected from LAG525 (Novartis), BMS-986016 (Bristol-Myers Squibb), or TSR-033 (Tesaro).

In addition, the present method can be used in a clinical trial analysis. Indeed, when applied to a population of patients, the analysis of the genes classified into the three classes and the therapeutic response to an immune checkpoint therapy may permit to define specific patterns of the genes classified into the three classes suitable for predicting a response to the treatment and for selecting the patients to be treated and/or those for whom the treatment is useless or to be avoided.

The present invention also relates to a method for selecting a set of genes for which the differential expression between a tumor sample and a normal histologically matched sample from the same patient is indicative of a therapeutic benefit of a treatment with an immune checkpoint blockade therapy. More particularly, as disclosed above, the method provides the mRNA expression level, and optionally miRNA expression levels, in a tumor sample and a normal histologically matched sample from the same patient.

This information is provided for a group of patients having a cancer and receiving, having received and planed to receive the same immune checkpoint blockade therapy. The group of patients may include 3, 4, 5, 6, 7, 8, 9, 10, 15, 20, 30, 40, or 50 patients or more. By the same immune checkpoint blockade therapy, it is intended that the immune checkpoint blockade therapy has the same target, i.e., PD-1/PD-L1, PD-L2 or CTLA-4/CD80,CD86,CD28. More specifically, the same immune checkpoint blockade therapy can be an antibody directed against the same protein, e.g., PD-1, PD-L1, CTLA-4, or LAG3. Still more specifically, the same immune checkpoint blockade therapy can be the same antibody. Optionally, the immune checkpoint blockade therapy is selected from the group consisting an anti-PD-1 antibody, an anti-PD-Ll antibody, an anti-CTLA-4 antibody, an anti-LAG3 antibody and any combination thereof.

Optionally, the patients may have any type of cancer or any type of solid tumors. Optionally, the patients may have the same type of cancers. Optionally, the patients may have the same cancer. Optionally, the patients may have various therapeutic history. Optionally, the patients may have received the same number of therapeutic lines, or even the same therapeutic lines.

The set of genes are as defined previously in the present disclosure. Then, the set of genes comprising genes of one or more of the following groups of genes: 1) PD-1, PD-L1, and PD-L2; 2) CTLA-4, CD80, CD86, and CD28; 3) LAG3; 4) TLR4 and 5) CD8, CD16 and FOXP3; the set of genes comprising at least the genes of the group 1) or 2). More specifically, the set of genes can be selected in one of the following sets: a) PD-1, PD-L1, PD-L2, CTLA-4, CD80, CD86, CD28, LAG3, TLR4, CD8, CD16 and FOXP3; b) PD-1, PD-L1, PD-L2, CTLA-4, CD80, CD86, LAG3 and TLR4; c) PD-1, PD-L1, PD-L2, CTLA-4, CD80, CD28, CD8, CD16 and FOXP3; d) PD-1, PD-L1, PD-L2, LAG3, TLR4, CD8, CD16 and FOXP3; and e) CTLA-4, CD80, CD86, CD28, LAG3, TLR4, CD8, CD16 and FOXP3. In a preferred aspect, the set of genes comprises, essentially consists in or consists in the genes PD-1, PD-L1, PD-L2, CTLA-4, CD80, CD86, CD28, LAG3, TLR4, CD8, CD16 and FOXP3. Optionally, the total number of genes is no more than 15 genes.

Then, based on the level of expression, the fold change of the mRNA expression between the tumor sample and the normal histologically matched sample (TvN fold change) of each gene and for each patient is determined. Optionally, the fold change of the mRNA expression can be corrected by taking into account the miRNA expression, especially of the miRNA as listed in Table 2.

The fold changes of each gene, optionally with the expression of the gene, respectively, can be used to determine the correlation between the fold change (with or without the expression) and the therapeutic benefit of the patient to the immune checkpoint blockade. The therapeutic benefit of the patient to the immune checkpoint blockade can be assessed based on any parameter usually used in clinical trials, such as OS (overall survival), tumor growth or regression, the disease-free survival, the relapse, the duration of the response, the disease progression, etc...

For instance, the correlation between the TvN fold changes for several combinations of genes and the therapeutic benefit can be determined and the combination(s) associated with the best correlation is/are selected. The step of correlation calculation can be computer-implemented step. The combination may include some or all combinations of 4, 5, 6, 7, 8, 9, 10, 11 and/or 12 genes of the set of genes.

On the basis of the selected combination, it is then provided methods for predicting the therapeutic benefit to the immune checkpoint blockade therapy of a subject, methods for selecting the subject who will have a therapeutic benefit of a treatment with the immune checkpoint blockade therapy, methods for determining that a subject is not a responder, methods for defining a sub-group of patients that is suitable for receiving a treatment with the immune checkpoint blockade therapy, etc...

Accordingly, the present disclosure provides a method for selecting a set of genes for which the differential expression between a tumor sample and a normal histologically matched sample from the same patient is indicative of a therapeutic benefit of a treatment with an immune checkpoint blockade therapy, wherein the method comprises
- providing, for several patients having a cancer and being treated with the same immune checkpoint blockade therapy, mRNA expression level in a tumor sample and a normal histologically matched sample of a set of genes comprising genes of one or more of the following groups of genes: 1) PD-1, PD-L1, and PD-L2; 2) CTLA-4, CD80, CD86, and CD28; 3) LAG3; 4) TLR4 and 5) CD8, CD16 and FOXP3; the set of genes comprising at least the genes of the group 1) or 2); and wherein the tumor and normal histologically matched samples are from the same subject;
- determining the correlation for the differential expression of combinations of 4, 5, 6, 7, 8, 9 and/or 10 genes of the set of genes; and,
- selecting the combination of genes associated with the best correlation to therapeutic benefit of a treatment with an immune checkpoint blockade therapy.

By applying the following method, the inventors identified correlation of interest.

Accordingly, the present disclosure provides a method for determining if a subject suffering from a cancer is susceptible to have a therapeutic benefit of a treatment with an immune checkpoint blockade therapy targeting CTLA-4, especially an anti-CTLA-4 antibody, wherein the expression level of a set of genes comprising or consisting of PD-1, PD-L1, CD80, CD28, LAG3, TLR4, CD8, CD16 and FOXP3 is determined in a tumor sample and a normal histologically matched sample from the patient, and the susceptibility for having a therapeutic benefit of a treatment with the immune checkpoint blockade therapy targeting CTLA-4, especially an anti-CTLA-4 antibody, is inversely correlated with the fold change of the expression for the set of genes.

In addition, the present disclosure provides a method for determining if a subject suffering from a cancer is susceptible to have a therapeutic benefit of a treatment with an immune checkpoint blockade therapy targeting PD-1/PD-L1, especially an anti-PD-1 or anti-PD-Ll antibody, wherein the expression level of a set of genes comprising or consisting of PD-L2, CTLA-4, LAG3, TLR4 and FOXP3 is determined in a tumor sample and a normal histologically matched sample from the patient, and the susceptibility for having a therapeutic benefit of a treatment with the immune checkpoint blockade therapy targeting PD-1/PD-L1, especially an anti-PD-1 or anti-PD-Ll antibody, is inversely correlated with the fold change of the expression for the set of genes.

### EXAMPLES

To provide consistent data supporting the new biomarker concept described in the invention, the inventors used the data obtained from patients treated in the clinical trial WINTHER (NCT01856296). The results of Winther trial were published in Nature Medicine volume25, pages 751-758 (2019). The inventors only used from this work the transcriptomics data. The methodology used to obtain transcriptomic data is provided in the Nature Medicine article, which incorporated herein by reference.

This is the first and as of today remains the only clinical trial that used transcriptomics in a clinical setting in addition to classic sequencing of DNA. It is also the first trial and the only that used the dual biopsies strategy investigating both tumor and normal matched tissue from the same patients, harboring a variety of solid tumors: NSCLC, Head & Neck, Colon, breast, bladder, liver, kidney, liposarcoma, rhabdomiosarcoma, neuroendocrine tumors, stomach, and oesophagus tumors. Based on DNA and RNA investigations, 107 patients could be treated in personalized manner with drugs selected among 159 different medicines. Patients were treated, and followed, and the clinical outcome (Progression free survival and overall survival could be recorded). The results of Winthertrial, published in Nature medicine, outline the benefit of using transcriptomics in a clinical setting.

Among the patients of Winther trial, 6 were treated with immunotherapies: 3 of them received ipilimumab (anti-CTLA-4) and 3 patients received anti-PD-L1/PD-1 based therapies: one patient received pembrolizumab, one patient received nivolumab, and one patient received atezolizumab. The overall survival under treatment was recorded for all of the 6 patients, and availability of transcriptomics data enabled to perform the novel digital display investigations described in this invention. These data as illustrated by Figure 2 show that simultaneously investigating the matched phenotypically normal tissue can help optimize transcriptomic data. With this approach, each patient serves as his or her own control, hence avoiding the use of pooled tumor or normal tissues. Our data demonstrate that the level of basal gene expression is highly variable between individuals. All patients presented with black points had no differential expression between tumor and normal, but others show a large variability between individuals in the basal level of normal expression of key players of immunoblockade.

Figures 3 and 4 illustrate Immune Checkpoint Blockade Digital Display for several patients and why it provides useful information in order to select the appropriate treatment for a subject.

From Figure 3, the Digital Display profile enables to assess the interaction between the key players: PD-L1 (and PD-L2) are significantly over expressed in tumors and therefore generates an efficient immune blockade on PD-1 receptor. Furthermore, CTLA-4 is also highly overexpressed in tumors and will therefore efficiently inhibit the co-stimulatory receptor CD28, through competitive binding of CD86 (B7-1). LAG3 is also scientifically over-expressed in tumors contributing to enforcement of negative Immune blockade. TLR4 is not different in tumors and normal tissues, in this example. There is a high level of infiltrating cytotoxic LyT (CD8+) and Natural Killer (NK cells) whilst T-regs are equally distributed in tumor and normal tissues. The Immune Checkpoint Blockade Digital Display gives physicians a visual representation of the status of the key players of the immune blockade and enables rapid interpretations and selection of appropriate therapeutic decisions. In this example, the patient should receive a combined Immune Checkpoint Blockade therapy against PD-1/PD-L1 and CTLA-4) (and ideally anti-LAG3). For example, the use of a monotherapy against PD-L1/PD-1 will have only a modest effect, and limited in duration, because the CTLA-4 and LAG3 negative blockade remains highly efficient.

Figure 4A: Example of patient with an Immune Checkpoint Blockade digital display showing there is no rationale for immunotherapy with anti-CTLA4 or anti-PDL1/PD1. Indeed, there is no differential expression between tumor and normal matched tissue. There is no activation of immune blockade in tumor, and therapy is not recommended. These profiles suggest that it would be no specific benefit for the tumor treatment, and may suggest also possibility of toxic effects.

Figure 4B: Example of a patients with an Immune Checkpoint Blockade digital display suggesting that immunotherapy with ant-CTLA4 or anti-PDL1/PD1 would be highly toxic, as both CTLA4 and PDL1 are under-expressed in tumor as compared with normal matched tissue.

The examples in Figure 5 illustrate and support the concept of the invention. Indeed, in patients treated with anti-CTL-4 therapy, the level of PD-L1 inversely correlates with the survival, whilst interaction of 8 genes perfectly correlates with the survival. Indeed, the prediction of outcome based only on analysis of CTLA-4 would not have correlate with clinical outcome. Therefore, to explain and, further to predict efficacy of treatment, it is necessary to take into account all key partners of the immunoblockade.

The examples in Figure 6 illustrate and support the concept of the inventions. Indeed, in patients treated with PD-1 therapy, the level of TLR4 inversely correlates with the survival, whilst interaction of 5 genes perfectly correlates with the survival. Indeed, the prediction of outcome based only on analysis of PD-L1/PD-1 would not have correlate with clinical outcome. Therefore, to explain and, further to predict efficacy of treatment, it is necessary to take into account all key partners of the immune blockade.

## Claims

1. A method for determining if a subject suffering from a cancer is susceptible to have a therapeutic benefit of a treatment with an immune checkpoint blockade therapy, comprising
- providing mRNA expression level in a tumor sample and a normal histologically matched sample of a set of genes comprising genes of one or more of the following groups of genes: 1) PD-1, PD-L1, and PD-L2; 2) CTLA-4, CD80, CD86, and CD28; 3) LAG3; 4) TLR4 and 5) CD8, CD16 and FOXP3; the set of genes comprising at least the genes of the group 1) or 2); and wherein the tumor and normal histologically matched samples are from the same subject; the mRNA expression level being optionally corrected with the expression of miRNA targeting the transcript;
- classifying the genes into three classes: i) a first class in which genes are overexpressed in the tumor sample in comparison to the normal histologically matched sample; ii) a second class in which gene are expressed at a similar level in the tumor sample in comparison to the normal histologically matched sample; and iii) a third class in which genes are underexpressed in the tumor sample in comparison to the normal histologically matched sample; and
- displaying the genes classified into the three classes.

2. The method according to claim 1, wherein the set of genes comprises at least the genes of the group 1) and 2).

3. The method according to claim 1 or 2, wherein the set of genes is selected in one of the following sets: a) PD-1, PD-L1, PD-L2, CTLA-4, CD80, CD86, CD28, LAG3, TLR4, CD8, CD16 and FOXP3; b) PD-1, PD-L1, PD-L2, CTLA-4, CD80, CD86, LAG3 and TLR4; c) PD-1, PD-L1, PD-L2, CTLA-4, CD80, CD28, CD8, CD16 and FOXP3; d) PD-1, PD-L1, PD-L2, LAG3, TLR4, CD8, CD16 and FOXP3; and e) CTLA-4, CD80, CD86, CD28, LAG3, TLR4, CD8, CD16 and FOXP3.

4. The method according to any one of claims 1-3, wherein mRNA expression level of other genes is studied in the method but the total number of genes is no more than 15 genes.

5. The method according to any one of claims 1-4, wherein a gene is overexpressed when the fold change between the tumor sample and the normal histologically matched sample is higher than 1.3, a gene is expressed at a similar level when the fold change is between -1.3 and 1.3, and a gene is underexpressed when the fold change is lower than -1.3.

6. The method according to any one of claims 1-5, wherein the genes classified into the three classes are displayed as a graph, preferably a graph showing the expression intensity in the tumor sample on the ordinate and the expression intensity in the normal histologically matched sample on the abscissa.

7. The method according to claim 6, wherein the genes of each of the three classes are shown with a distinct mode between each other's and optionally the genes of each of the groups are also shown with a distinct mode between each other's.

8. The method according to any one of claims 1-7, wherein the susceptibility to have a therapeutic benefit of a treatment with an immune checkpoint blockade therapy is assessed based on the presence of genes of the groups in one of the three classes and optionally the expression intensity of the genes of the groups in the tumor sample.

9. The method according to any one of claims 1-8, wherein the immune checkpoint blockade therapy is selected from the group consisting an anti-PD-1 antibody, an anti-PD-Ll antibody, an anti-CTLA-4 antibody, an anti-LAG3 antibody and any combination thereof.

10. A method for selecting a set of genes for which the differential expression between a tumor sample and a normal histologically matched sample from the same patient is indicative of a therapeutic benefit of a treatment with an immune checkpoint blockade therapy, wherein the method comprises
- providing, for several patients having a cancer and being treated with the same immune checkpoint blockade therapy, mRNA expression level in a tumor sample and a normal histologically matched sample of a set of genes comprising genes of one or more of the following groups of genes: 1) PD-1, PD-L1, and PD-L2; 2) CTLA-4, CD80, CD86, and CD28; 3) LAG3; 4) TLR4 and 5) CD8, CD16 and FOXP3; the set of genes comprising at least the genes of the group 1) or 2); and wherein the tumor and normal histologically matched samples are from the same subject; the mRNA expression level being optionally corrected with the expression of miRNA targeting the transcript;
- determining the correlation for the differential expression of combinations of 4, 5, 6, 7, 8, 9 and/or 10 genes of the set of genes; and,
- selecting the combination of genes associated with the best correlation to therapeutic benefit of a treatment with an immune checkpoint blockade therapy.

11. The method according to claim 10, wherein the set of genes is selected in one of the following sets: a) PD-1, PD-L1, PD-L2, CTLA-4, CD80, CD86, CD28, LAG3, TLR4, CD8, CD16 and FOXP3; b) PD-1, PD-L1, PD-L2, CTLA-4, CD80, CD86, LAG3 and TLR4; c) PD-1, PD-L1, PD-L2, CTLA-4, CD80, CD28, CD8, CD16 and FOXP3; d) PD-1, PD-L1, PD-L2, LAG3, TLR4, CD8, CD16 and FOXP3; and e) CTLA-4, CD80, CD86, CD28, LAG3, TLR4, CD8, CD16 and FOXP3, preferably PD-1, PD-L1, PD-L2, CTLA-4, CD80, CD86, CD28, LAG3, TLR4, CD8, CD16 and FOXP3.

12. The method according to any one of claims 10-11, wherein mRNA expression level of other genes is studied in the method but the total number of genes is no more than 15 genes.

13. The method according to any one of claims 10-12, wherein the immune checkpoint blockade therapy is selected from the group consisting an anti-PD-1 antibody, an anti-PD-Ll antibody, an anti-CTLA-4 antibody, an anti-LAG3 antibody and any combination thereof.

14. A method for determining if a subject suffering from a cancer is susceptible to have a therapeutic benefit of a treatment with an immune checkpoint blockade therapy targeting CTLA-4, especially an anti-CTLA-4 antibody, wherein the expression level of a set of genes comprising or consisting of PD-1, PD-L1, CD80, CD28, LAG3, TLR4, CD8, CD16 and FOXP3 is determined in a tumor sample and a normal histologically matched sample from the patient, and the susceptibility for having a therapeutic benefit of a treatment with the immune checkpoint blockade therapy targeting CTLA-4, especially an anti-CTLA-4 antibody, is inversely correlated with the fold change of the expression for the set of genes.

15. A method for determining if a subject suffering from a cancer is susceptible to have a therapeutic benefit of a treatment with an immune checkpoint blockade therapy targeting PD-1/PD-L1, especially an anti-PD-1 or anti-PD-Ll antibody, wherein the expression level of a set of genes comprising or consisting of PD-L2, CTLA-4, LAG3, TLR4 and FOXP3 is determined in a tumor sample and a normal histologically matched sample from the patient, and the susceptibility for having a therapeutic benefit of a treatment with the immune checkpoint blockade therapy targeting PD-1/PD-L1, especially an anti-PD-1 or anti-PD-Ll antibody, is inversely correlated with the fold change of the expression for the set of genes.
